# EUROPÄISCHE PATENTSCHRIFT

(11) **EP 1 488 751 B1**
(45) Veröffentlichungstag und Bekanntmachung des Hinweises auf die Patenterteilung: **04.11.2009**
(21) Anmeldenummer: 04009759.4
(22) Anmeldetag: 24.04.2004
(51) Int. Cl.: A61B 17/70

(54) **Implantat zur Korrektur und Stabilisierung der Wirbelsäule**
Implant for stabilisation and correction of the spine
Implant pour la stabilisation et la correction du rachis

(30) Priorität: 16.06.2003 DE 10327358
(43) Veröffentlichungstag der Anmeldung: 22.12.2004
(73) Patentinhaber: Ulrich GmbH & Co. KG, 89081 Ulm (DE)
(72) Erfinder: Sutcliffe, John, Good Easter, Essex CM 1 4 PR (GB); Richter, Marcus, 89275 Elchingen (DE); Mack, Thomas, 89160 Dornstadt (DE); Willmann, Nikolas, 89077 Ulm (DE)
(74) Vertreter: Hentrich, Swen

(56) Entgegenhaltungen:
- EP-A- 1 281 364
- WO-A-2004/024011
- WO-A2-2004/105577
- FR-A- 2 718 946
- FR-A- 2 726 995
- FR-A- 2 799 949
- GB-A- 2 382 304

## Beschreibung

Die Erfindung betrifft ein Implantat zur Korrektur und Stabilisierung der Wirbelsäule, bestehend aus in die Wirbel eindrehbaren Pedikelschrauben und aus mindestens einem die Pedikelschrauben an den Schraubenköpfen verbindenden Verbindungselement, wobei das Verbindungselement durch eine Spirale gebildet ist, deren Spiralwindungen einer Schraubenlinie folgend in axialer Richtung versetzt angeordnet sind, und die Spirale zweifach vorgesehen und die eine der Spiralen im Spiralinneren der anderen Spirale angeordnet ist, wobei im Spiralinneren der inneren Spirale mindestens über einen Teilbereich ihrer axialen Erstreckung ein Spiralkern angeordnet ist, der als Stange gebildet ist.

Ein Implantat zur Korrektur und Stabilisierung der Wirbelsäule ist beispielsweise in der DE 41 10 002 C1 beschrieben. Bei diesem Implantat ist das Verbindungselement durch einen Metallstab gebildet, der mit den Pedikelschrauben über die Länge mindestens zweier benachbarter Wirbel an der Wirbelsäule fixiert wird, um diese zu korrigieren und zu stabilisieren, das heißt die Wirbel in eine bestimmte räumliche Lage zu zwingen und dort festzulegen. Damit ist eine bereichsweise Versteifung der Wirbelsäule verbunden, die gewährleistet, daß die Wirbelsäule ihre tragende Funktion ausüben kann, auch wenn einzelne Wirbel oder Bandscheiben im Zwischenwirbelraum traumatisch geschädigt oder degenerativ erkrankt sind. In dem durch das Implantat versteiften Bereich der Wirbelsäule ist die natürliche Beweglichkeit einer gesunden Wirbelsäule unterbunden, so daß benachbarte Bereiche der Wirbelsäule stärker belastet werden und damit anfälliger sind für Abnutzung und Verschleiß, also erneuten degenerativen Erkrankungen.

Die nach-veröffentlichte WO-A-2004/105577 offenbart ein Implantat zur Korrektur und Stabilisierung der Wirbelsäule nach dem Oberbegriff des Anspruches 1.

Der Erfindung liegt die Aufgabe zugrunde, ein Implantat der eingangs genannten Art so auzubilden, daß nach dessen Implantation eine starke Annäherung an die physiologischen Bewegungsmöglichkeiten einer gesunden Wirbelsäule gegeben ist.

Diese Aufgabe wird nach der Erfindung bei einem Implantat zur Korrektur und Stabilisierung der Wirbelsäule nach dem Oberbegriff des Anspruches 1 dadurch gelöst, daß die Stange mehrteilig aus mehreren Stangenabschnitten gebildet ist, die Unterschiede in ihren Materialeigenschaften aufweisen.

Mit dieser Gestaltung ist der Vorteil verbunden, daß das Verbindungselement weiterhin eine Stabilisierung der Wirbelsäule bewirkt, daß zugleich aber aufgrund der aus der Form des Verbindungselementes sich intrinsisch ergebenden Federeigenschaften eine begrenzte Beweglichkeit der Wirbelsäule auch in dem stabilisierten Bereich bereitgestellt wird, die eine bessere physiologische Anpassung des stabilisierten Bereiches an die gesunden Nachbarbereiche ermöglicht. Insbesondere auch bei impulsartigen Krafteinwirkungen auf den stabilisierten Bereich kann dieser in Folge des als Spirale gestalteten Verbindungselementes durch Auslenkung der Spirale die Kräfte aufnehmen und nachfolgend nach deren Ableitung über ausgedehntere Bereiche der Wirbelsäule wieder in seine Ruhelage zurückkehren.

Der Spiralkern wird dazu genutzt, die Federkonstante beziehungsweise die Biegesteifigkeit der Spirale zu beeinflussen, so daß sich beispielsweise die Möglichkeit ergibt, die Spirale sich über den Bereich mehrerer Wirbel erstrecken zu lassen, wobei lediglich in einem begrenzten Gebiet durch den Einsatz des Spiralkerns eine erhöhte Steifigkeit der Spirale bereit gestellt wird.

Da die Stange mehrteilig aus mehreren Stangenabschnitten gebildet ist, die Unterschiede in ihren Materialeigenschaften, insbesondere in ihrer Biegesteifigkeit aufweisen, ergibt sich die Möglichkeit, über die axiale Ausdehnung der Spirale unterschiedliche, auch aus unterschiedlichen Materialien wie Metall oder Kunststoff gefertigte Spiralkerne zu verwenden und so lokal die sich aus Form und Material der Spirale ergebenden Eigenschaften, insbesondere die Biegesteifigkeit zu modifizieren.

Durch die Ausführungsform ist eine Vereinfachung der Handhabung des Verbindungselementes während der Operation gegeben, wobei wiederum eine Veränderung der Biegesteifigkeit der isolierten ersten Spirale erreicht wird.

Für das einfache Einsetzen der zweiten Spirale in das Spiralinnere der ersten Spirale hat es sich als zweckmäßig erwiesen, wenn der Drehsinn der Wickelung der beiden Spiralen gegenläufig ist, wobei bei dieser Ausführungsform auch die Federwirkung der beiden Spiralen erhalten bleibt und nicht die Gefahr besteht, daß die beiden Spiralen mit ihren Wicklungen sich zu einem Pseudo-Zylinder ergänzen.

Für die einfache Verbindung der Spirale mit den Pedikelschrauben ist es vorgesehen, daß den Schraubköpfen der Pedikelschrauben zum Einlegen der Spirale Aufnahmen zugeordnet sind, die Klemmmittel zum Sichern der Spirale in den Aufnahmen aufweisen, wobei bei der erfindungsgemäßen Gestaltung der Spiralen die Möglichkeit besteht, diese nicht lediglich reibschlüssig in den Aufnahmen mit den Klemmmitteln zu sichern, sondern auch verwirklicht werden kann, daß die Klemmmittel durch Klemmschrauben gebildet sind, die durch zwischen zwei benachbarte Spiralwindungen formschlüssig eingreifen.

Als vorteilhaft hat es sich weiterhin erwiesen, wenn bei einer Spirale für die vorstehend genannten Implantate die Spirale als Flachdrahtspirale gebildet ist. Gegegenüber einem Rundmaterial bietet sich der Vorteil, daß bei vergleichbar geringem Durchmesser der Spirale eine größere axiale Erstreckung der Spirale erreicht ist, die ihrer Funktion als Verbindungselement zwischen Pedikelschrauben entgegenkommt. Angesichts des angestrebten Verwendungszweckes bietet es sich an, daß die Spirale aus Titan oder chirurgischem Stahl gebildet ist.

Um die komplexe und vielfältige Funktion des Implantates mit der Spirale hinsichtlich einer ausreichenden Stabilisierung der Wirbelsäule bei zugleicher Bereitstellung einer gewissen Beweglichkeit zu erzielen, hat es sich als günstig erwiesen, wenn der Flachdraht eine Materialdicke von 0,4 mm bis 2,8 mm, vorzugsweise von 1,2 mm aufweist. Zur Förderung der Funktion der Spirale ist es weiterhin günstig, wenn der Flachdraht eine Materialbreite von 2 mm bis 8 mm, vorzugsweise von 4 mm bis 6 mm aufweist. Hinsichtlich des Innendurchmessers sind die Abmessungen so gewählt, daß der Innendurchmesser eine Größe von 2,5 mm bis 7,5 mm, vorzugsweise von 3,2 mm bis 4,6 mm aufweist. Um eine Dämpfung bereitzustellen, wenn eine Belastung der Spirale in ihrer axialen Richtung erfolgt, weisen benachbarte Spiralwindungen einen Abstand von 0,5 mm bis 2,5 mm auf.

Bei einer Spirale zur Verwendung im Spiralinneren der äußeren Spirale ist vorgesehen, daß der Abstand benachbarter Spiralwindungen größer als bei der äußeren Spirale gewählt ist, wodurch auch deutlich wird, daß die zweite Spirale lediglich zur Unterstützung der äußeren Spirale vorgesehen ist.

Im folgenden wird die Erfindung an in der Zeichnung dargestellten Ausführungsbeispielen näher erläutert; es zeigen:
- Fig. 1: eine schematische Darstellung zweier symmetrisch zur Längsachse der Wirbelsäule an dieser befestigten Implantate,
- Fig. 2: eine perspektivische, isolierte Darstellung des Verbindungselementes des Implantats aus Fig. 1,
- Fig. 3: das Detail III aus Fig. 2,
- Fig. 4: eine der Fig. 2 entsprechende Darstellung eines Verbindungselementes gemäß der Erfindung mit einem in die innere Spirale eingesetzten Spiralkern,
- Fig. 5: das Detail V aus Fig. 4,
- Fig. 6: eine Seitenansicht des Verbindungselementes aus Fig. 4,
- Fig. 7: eine der Fig. 6 entsprechende Darstellung der als Flachdrahtspirale gebildeten äußeren Spirale,
- Fig. 8: eine der Fig. 6 entsprechende Darstellung der als Flachdrahtspirale gebildeten inneren Spirale, und
- Fig. 9: eine Seitenansicht des mehrteilig gebildeten Spiralkerns.

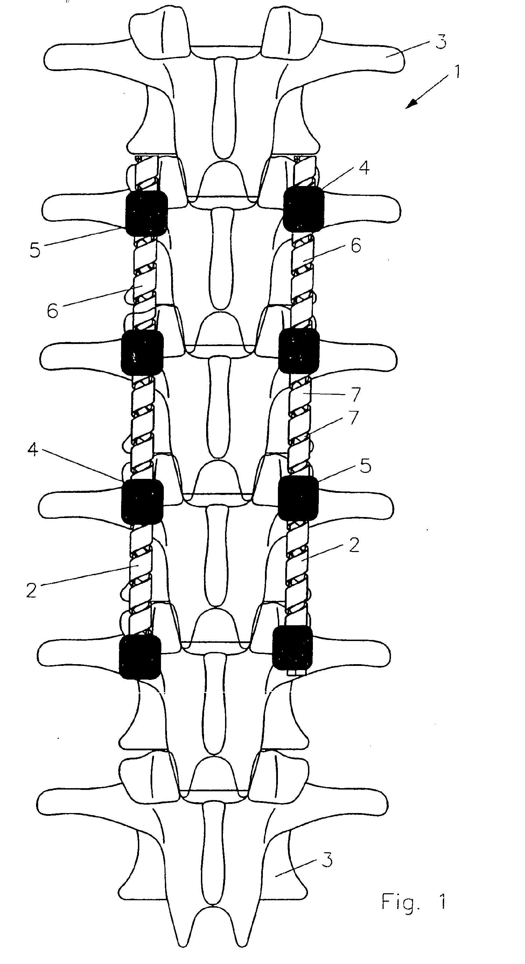

In der Fig. 1 ist eine Wirbelsäule 1 dargestellt, die in ihrer Funktion mittels zweier Implantate 2 unterstützt wird, die der Korrektur und der Stabilisierung der Wirbelsäule 1 dienen, also dabei helfen, die einzelnen Wirbel 3 der Wirbelsäule 1 in ihren anatomisch richtigen Positionen zu halten. Jedes Implantat 2 besteht aus mehreren, in dem gezeigten Ausführungsbeispiel aus vier Pedikelschrauben 4, sowie aus einem die Pedikelschrauben 4 an den Schraubenköpfen 5 verbindenden Verbindungselement 6. Das Verbindungselement 6 ist durch eine Spirale 7 gebildet, die ihrerseits als Flachdrahtspirale gestaltet ist, deren Spiralwindungen einer Schraubenlinie folgend in axialer Richtung versetzt angeordnet sind, wobei benachbarte Spiralwindungen einen Abstand von 0,5 mm bis 2,5 mm aufweisen. In der Zeichnung ist ein Ausführungsbeispiel dargestellt, bei dem für den Flachdraht aus einem Bereich von 0,4 mm bis 2,8 mm eine Materialdicke von 1,2 mm gewählt ist, sowie eine Materialbreite von 6 mm aus einem Bereich von 2 mm bis 8 mm. Der Durchmesser der Wicklung, also der Innendurchmesser der Spirale 7 liegt im Bereich von 2,5 mm bis 7,5 mm.

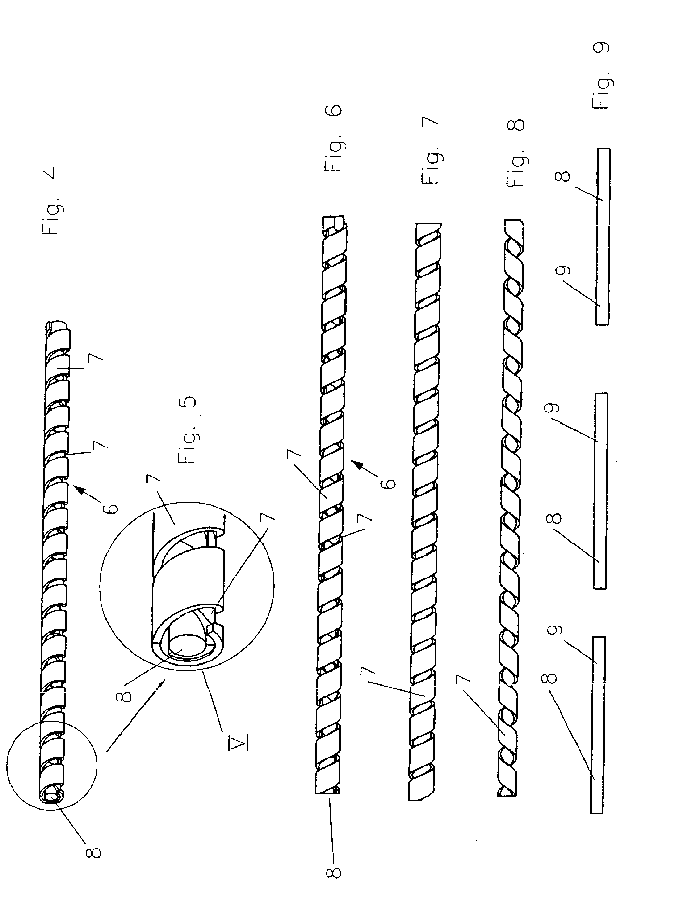

Bei den in den Figuren 5 bis 9 dargestellten Ausführungsbeispiel ist im Inneren der Spirale 7 mindestens über einen Teilbereich ihrer axialen Erstreckung ein Spiralkern 8 angeordnet, der als Stange ausgebildet ist, die ihrerseits mehrteilig aus mehreren Stangenabschnitten 9 besteht, die Unterschiede in ihren Materialeigenschaften, insbesondere in ihrer Biegesteifigkeit aufweisen. Beispielsweise kann die in der Fig. 9 dargestellte Stange als Spiralkern 8 mit ihrem mittleren Stangenabschnitt 9 aus Metall, insbesondere Titan oder chirurgischem Stahl gebildet sein, während die äußeren Stangenabschnitte 9 aus Kunststoff bestehen. Damit ergibt sich die Möglichkeit, die Eigenschaften des Verbindungselementes 6 und des Implantates 2 insgesamt zu variieren und so Bereiche größerer Steifheit beziehungsweise größerer Beweglichkeit durch geeignete Wahl des Spiralkerns 8 zu schaffen, auch wenn die grundsätzliche Festlegung der Basiseigenschaften durch die Dimensionierung des Flachdrahtes bereits erfolgt ist.

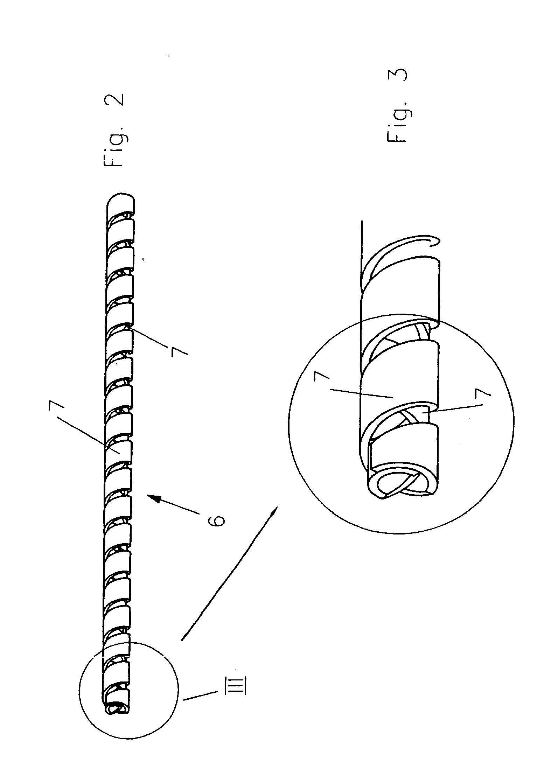

Sowohl das Ausführungsbeispiel in den Figuren 2 und 3 als auch in den Figuren 5 bis 9 zeigt eine Konstellation, bei der die Spirale 7 zweifach vorgesehen und die eine der Spiralen 7 im Spiralinneren der anderen Spirale 7 angeordnet ist, wobei der Drehsinn der Wicklungen der beiden Spiralen 7 gegenläufig ist. Aufgrund der grundsätzlich gleichen Gestaltung der äußeren und der inneren Spirale 7 ergibt sich auch die Möglichkeit, den Spiralkern 8 im Spiralinneren der inneren Spirale 7 anzuordnen.

Die in der Zeichnung in Fig. 1 nur schematisch dargestellten Pedikelschrauben 4 weisen ihren Schraubenköpfen 5 zugeordnete Aufnahmen auf, denen Klemmmittel zum Sichern der Spirale 7 in den Aufnahmen zugeordnet sind, nämlich Klemmschrauben, die zwischen zwei benachbarte Spiralwindungen formschlüssig eingreifen, so daß eine verbesserte Sicherung der Spirale 7, also des Verbindungselements 6 zwischen zwei Pedikelschrauben 4 gegen Verschiebung in Axialrichtung gegeben ist.

### Bezugszeichenliste

- 1: Wirbelsäule
- 2: Implantat
- 3: Wirbel
- 4: Pedikelschraube
- 5: Schraubenkopf
- 6: Verbindungselement
- 7: Spirale
- 8: Spiralkern
- 9: Stangenabschnitt

## Patentansprüche

1. Implantat zur Korrektur und Stabilisierung der Wirbelsäule (1), bestehend aus in die Wirbel (3) eindrehbaren Pedikelschrauben (4) und aus mindestens einem die Pedikelschrauben (4) an den Schraubenköpfen (5) verbindenden Verbindungselement (6), wobei das Verbindungselement (6) durch eine Spirale (7) gebildet ist, deren Spiralwindungen einer Schraubenlinie folgend in axialer Richtung versetzt angeordnet sind, und die Spirale (7) zweifach vorgesehen und die eine der Spiralen (7) im Spiralinneren der anderen Spirale (7) angeordnet ist, wobei im Spiralinneren der inneren Spirale (7) mindestens über einen Teilbereich ihrer axialen Erstreckung ein Spiralkern (8) angeordnet ist, der als Stange gebildet ist, **dadurch gekennzeichnet, dass** die Stange mehrteilig aus mehreren Stangenabschnitten (9) gebildet ist, die Unterschiede in ihren Materialeigenschaften aufweisen.

2. Implantat nach Anspruch 1, **dadurch gekennzeichnet, daß** die Stange aus Metall, insbesondere Titan oder chirurgischen Stahl, gebildet ist.

3. Implantat nach Anspruch 1 oder 2, **dadurch gekennzeichnet, daß** der Drehsinn der Wickelungen der beiden Spiralen (7) gegenläufig ist.

4. Implantat nach einem der Ansprüche 1 bis 3, **dadurch gekennzeichnet, daß** den Schraubenköpfen (5) der Pedikelschrauben (4) zum Einlegen der Spirale (7) Aufnahmen zugeordnet sind, die Klemmmittel zum Sichern der Spirale (7) in den Aufnahmen aufweisen.

5. Implantat nach Anspruch 4, **dadurch gekennzeichnet, daß** die Klemmmittel durch Klemmschrauben gebildet sind, die zwischen zwei benachbarte Spiralwindungen formschlüssig eingreifen.

6. Implantat nach einem der Ansprüche 1 bis 5, **dadurch gekennzeichnet, daß** die Spirale (7) als Flachdrahtspirale gebildet ist.

7. Implantat nach Anspruch 6, **dadurch gekennzeichnet, daß** die Spirale (7) aus Titan oder chirurgischen Stahl gebildet ist.

8. Implantat nach Anspruch 7, **dadurch gekennzeichnet, daß** der Flachdraht eine Materialdicke von 0,4 mm bis 2,8 mm, vorzugsweise von 1,2 mm aufweist.

9. Implantat nach Anspruch 7 oder 8, **dadurch gekennzeichnet, daß** der Flachdraht eine Materialbreite von 2 mm bis 8 mm, vorzugsweise von 4 mm bis 6 mm aufweist.

10. Implantat nach einem der Ansprüche 7 bis 9, **dadurch gekennzeichnet, daß** die Spirale (7) einen Innendurchmesser von 2,5 mm bis 7,5 mm, vorzugsweise von 3,2 mm bis 4,6 mm aufweist.

11. Implantat nach einem der Ansprüche 7 bis 10, **dadurch gekennzeichnet, daß** benachbarte Spiralwindungen einen Abstand von 0,5 mm bis 2,5 mm aufweisen.

## Claims

1. An implant for correction and stabilisation of the spinal column (1) comprising pedicle screws (4) which can be screwed into the vertebrae (3) and at least one connecting element (6) connecting the pedicle screws (4) to the screw heads (5), wherein the connecting element (6) is formed by a spiral (7), the spiral turns of which are arranged displaced in the axial direction and following a helical line, and the spiral (7) is provided in duplicate and the one of the spirals (7) is arranged in the interior of the other spiral (7), wherein arranged in the interior of the inner spiral (7) at least over a partial region of the axial extent thereof is a spiral core (8) in the form of a rod, **characterised in that** the rod is formed in a multi-part structure from a plurality of rod portions (9) which involve differences in their material properties.

2. An implant according to claim 1 **characterised in that** the rod is formed from metal, in particular titanium or surgical steel.

3. An implant according to claim 1 or claim 2 **characterised in that** the rotational direction of the turns of the two spirals (7) is in opposite relationship.

4. An implant according to one of claims 1 to 3 **characterised in that** receiving means are associated with the screw heads (5) of the pedicle screws (4) for insertion of the spiral (7), the receiving means having clamping means for securing the spiral (7) in the receiving means.

5. An implant according to claim 4 **characterised in that** the clamping means are formed by clamping screws which engage in positively locking relationship between two adjacent spiral turns.

6. An implant according to one of claims 1 to 5 **characterised in that** the spiral (7) is in the form of a flat wire spiral.

7. An implant according to claim 6 **characterised in that** the spiral (7) is formed from titanium or surgical steel.

8. An implant according to claim 7 **characterised in that** the flat wire is of a material thickness of 0.4 mm to 2.8 mm, preferably of 1.2 mm.

9. An implant according to claim 7 or claim 8 **characterised in that** the flat wire is of a material width of 2 mm to 8 mm, preferably 4 mm to 6 mm.

10. An implant according to one of claims 7 to 9 **characterised in that** the spiral (7) has an inside diameter of 2.5 mm to 7.5 mm, preferably 3.2 mm to 4.6 mm.

11. An implant according to one of claims 7 to 10 **characterised in that** adjacent spiral turns are at a spacing of 0.5 mm to 2.5 mm.

## Revendications

1. Implant pour corriger et stabiliser le rachis (1), constitué de vis pédiculaires (4), pouvant être insérées dans les vertèbres (3), et d'au moins un élément de liaison (6) reliant les vis pédiculaires (4) au niveau des têtes de vis (5), l'élément de liaison (6) étant formé d'une spirale (7) dont les tours sont décalés dans le sens axial en suivant une hélice, et la spirale (7) étant prévue en double, et l'une des spirales (7) étant disposée à l'intérieur de l'autre spirale (7), un noyau de spirale (8), réalisé sous forme de tige, étant placé à l'intérieur de la spirale interne (7), au moins sur une partie de sa dimension axiale, **caractérisé par le fait que** la tige est constituée de plusieurs tronçons de tige (9) qui présentent des différences au ni veau de leurs propriétés de matière.

2. Implant selon la revendication 1, **caractérisé par le fait que** la tige est réalisée en métal, notamment en titane ou en acier chirurgical.

3. Implant selon la revendication 1 ou 2, **caractérisé par le fait que** les enroulements des deux spirales (7) ont des sens de rotation opposés.

4. Implant selon une des revendications 1 à 3, **caractérisé par le fait que** sont associés aux têtes de vis (5) des vis pédiculaires (4), des logements qui sont destinés à recevoir la spirale (7) et qui présentent des moyens de serrage pour fixer la spirale (7) dans les logements.

5. Implant selon la revendication 4, **caractérisé par le fait que** les moyens de serrage sont constitués de vis de serrage qui s'engagent avec complémentarité de forme entre deux tours de spirale contigus.

6. Implant selon une des revendications 1 à 5, **caractérisé par le fait que** la spirale (7) est réalisée sous forme de spirale en fil métallique plat.

7. Implant selon la revendication 6, **caractérisé par le fait que** la spirale (7) est réalisée en titane ou en acier chirurgical.

8. Implant selon la revendication 7, **caractérisé par le fait que** le fil métallique plat présente une épaisseur de matière comprise entre 0,4 mm et 2,8 mm, de préférence de 1,2 mm.

9. Implant selon la revendication 7 ou 8, **caractérisé par le fait que** le fil métallique plat présente une largeur de matière comprise entre 2 mm et 8 mm, de préférence entre 4 mm et 6 mm.

10. Implant selon une des revendications 7 à 9, **caractérisé par le fait que** la spirale (7) présente un diamètre intérieur compris entre 2,5 mm et 7,5 mm, de préférence entre 3,2 mm et 4,6 mm.

11. Implant selon une des revendications 7 à 10, **caractérisé par le fait que** des tours de spirale contigus présentent un espacement compris entre 0,5 mm et 2,5 mm.
